(19) Europäisches Patentamt
European Patent Office
Office européen des brevets

(11) EP 1 500 387 B1

(12) **FASCICULE DE BREVET EUROPEEN**

(45) Date de publication et mention de la délivrance du brevet:
**09.01.2008 Bulletin 2008/02**

(51) Int Cl.:
***A61K 8/35*** $^{(2006.01)}$ ***A61Q 5/10*** $^{(2006.01)}$
***A61K 8/49*** $^{(2006.01)}$ ***A61K 8/40*** $^{(2006.01)}$
***A61K 8/41*** $^{(2006.01)}$

(21) Numéro de dépôt: **04291875.5**

(22) Date de dépôt: **23.07.2004**

(54) **Utilisation en coloration de dérivés de ninhydrine**

Verwendung von Ninhydrin-Derivaten in Färbemitteln

Use of ninhydrin derivatives for dyeing

(84) Etats contractants désignés:
**AT BE BG CH CY CZ DE DK EE ES FI FR GB GR HU IE IT LI LU MC NL PL PT RO SE SI SK TR**

(30) Priorité: **25.07.2003 FR 0309171**
**04.03.2004 FR 0402247**

(43) Date de publication de la demande:
**26.01.2005 Bulletin 2005/04**

(73) Titulaire: **L'ORÉAL**
**75008 Paris (FR)**

(72) Inventeurs:
- **Plos, Grégory**
**Tokyo 158-0097 (JP)**
- **Gourlaouen, Luc**
**92600 Asnieres (FR)**

(74) Mandataire: **Dossmann, Gérard**
**Bureau Casalonga & Josse**
**Bayerstrasse 71/73**
**80335 München (DE)**

(56) Documents cités:
**DE-A- 4 317 855**
**DE-A- 4 335 627**
**DE-A- 19 745 355**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne des compositions de coloration des matières kératiniques, en particulier des compositions de coloration capillaire contenant au moins un dérivé de ninhydrine associé, de préférence, à un composé à fonction amine primaire ou secondaire ou à un composé à fonction méthylène activée, un procédé de coloration utilisant de telles compositions et un agent de coloration multi-composants servant à la mise en oeuvre d'un tel procédé.

Depuis longtemps, de nombreuses personnes cherchent à modifier la couleur de leur peau, de leurs cils ou de leurs cheveux et en particulier à masquer leurs cheveux blancs. Pour ce faire, plusieurs technologies ont été développées.

Il est connu de teindre les fibres kératiniques humaines, et en particulier les cheveux, avec des compositions tinctoriales contenant des précurseurs de colorants d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. Ces colorants sont insolubles et sont piégés à l'intérieur de la fibre capillaire.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Les colorations obtenues présentent une bonne ténacité aux shampooings. Cependant, la réaction d'oxydation se fait à l'aide de produits oxydants tels l'eau oxygénée en milieu basique. Ces agents oxydants attaquent la kératine des cheveux dont les propriétés cosmétiques et mécaniques peuvent se dégrader fortement en cas de colorations répétées.

Il est aussi connu de teindre les fibres kératiniques humaines par une coloration directe consistant à appliquer sur les fibres kératiniques des colorants directs qui sont des molécules colorées et colorantes ayant une affinité pour les fibres. On peut citer à titre d'exemples de colorants directs utilisés classiquement les colorants nitrés, benzéniques, anthraquinoniques, nitropyridiniques, azoïques, azoïques cationiques, xanthéniques, acridiniques, aziniques ou de type triarylméthane ou des colorants naturels.

Les colorations obtenues ainsi sont, certes, très chromatiques et n'entraînent pas une dégradation chimique de la kératine, mais présentent l'inconvénient de n'être que temporaires ou semi-permanentes, c'est-à-dire de s'estomper après seulement 4 à 5 shampooings.

Il subsiste par conséquent un besoin de systèmes et de procédés de coloration qui permettent l'obtention de bonnes ténacités sans impliquer l'utilisation d'agents oxydants susceptibles de dégrader les matières kératiniques.

La demanderesse a découvert avec surprise que l'utilisation de dérivés particuliers de ninhydrine décrits plus en détail ci-dessous permettait de colorer les matières kératiniques, et en particulier les cheveux, avec des ténacités équivalentes voire supérieures à celles obtenues par coloration d'oxydation et ceci en l'absence d'agents oxydants forts préservant ainsi parfaitement les matières kératiniques.

Les dérivés de ninhydrine mentionnés ci-dessus sont utilisés de préférence en combinaison avec des composés à hydrogène labile, tels que des amines primaires ou secondaires ou des composés à fonction méthylène activée.

Les colorations obtenues ainsi présentent de bonnes chromaticités et se distinguent en particulier par une excellente ténacité au lavage (plusieurs dizaines de shampooings).

L'invention a donc pour objet l'utilisation pour la coloration de matières kératiniques, d'une composition contenant, dans un milieu approprié pour la teinture, au moins un composé de formule (I)

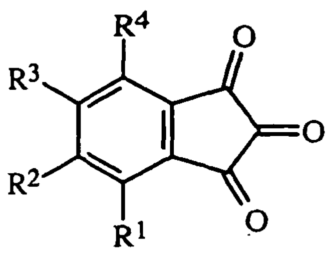

(I)

dans laquelle

$R^1$, $R^2$, $R^3$ et $R^4$ représentent, indépendamment les uns des autres, un atome d'hydrogène, un groupe carboxy (alkyle en $C_{1-6}$), (alkyle en $C_{1-6}$)-carboxy-(alkyle en $C_{1-6}$), aryloxy à 6 chaînons, hétéroaryloxy à 5 chaînons contenant un ou plusieurs hétéroatomes choisis parmi N, O, S et P, aryle comportant au moins 5 chaînons, monocyclique ou polycyclique, condensé ou non-condensé, contenant éventuellement un ou plusieurs hétéroatomes choisis parmi

N, O, S et P, les groupes aryloxy, hétéroaryloxy, aryle ou hétéroaryle ci-dessus portant éventuellement un ou plusieurs substituants choisis parmi les atomes d'halogène, les groupes alkyle en $C_{1-9}$, hydroxy, alcoxy en $C_{1-6}$, amino, mono- ou di-(alkyle en $C_{1-6}$)-amino, mono- ou di-(hydroxyalkyle en $C_{1-6}$)-amino, thio, alkylthio en $C_{1-6}$, thioalkyle en $C_{1-6}$, (alkyle en $C_{1-6}$)-carbonyle, hydrogénocarbo-nyle, hydroxycarbonyle, (alcoxy en $C_{1-6}$)-carbonyle, nitro, sulfonato, tri(alkyle en $C_{1-6}$)-ammonio, imidazolyle et pyridinyle, ou les groupes protonés correspondants tels que ammonio, imidazolio ou pyridinio,
ou deux substituants voisins représentent ensemble un groupe $-O-CH_2-O-$,
avec la réserve qu'au moins un des symboles $R^1$ à $R^4$ représente un groupe différent d'un atome d'hydrogène, ou $R^1$ et $R^2$, $R^2$ et $R^3$ ou $R^3$ et $R^4$ représentent, ensemble, un groupe $-O-CH_2-O-$.

De telles compositions sont en particulier utiles pour la teinture des fibres kératiniques, notamment les cheveux.
Les dérivés de ninhydrine de formule (I) ci-dessus sont utilisés dans la présente invention dans un milieu cosmétiquement acceptable contenant généralement une fraction importante d'eau. Lorsqu'ils sont dissous dans un tel milieu aqueux, les dérivés de ninhydrine de formule (I) sont en équilibre d'hydratation avec la forme diol géminé (ou hydrate de carbonyle) correspondant à la formule (I)bis suivante :

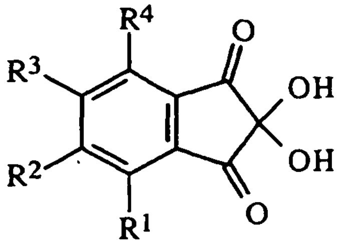

(I)bis

Lorsqu'il est question, dans la présente demande, de dérivés de ninhydrine de formule (I) ceux-ci englobent par conséquent toujours non seulement les composés de formule (I) mais également les formes hydratées correspondantes de formule (I)bis.
Lorsque les symboles $R^1$, $R^2$, $R^3$ ou $R^4$ représentent un noyau aryle, hétéroaryle, aryloxy ou hétéroaryloxy, les substituants de ces noyaux sont de préférence choisis de manière à ce que ce noyau forme, avec le noyau indane, un système à électrons $\pi$ délocalisés. De tels systèmes donnent en effets des colorations ayant des chromaticités particulièrement intéressantes.
Des exemples préférés de dérivés de ninhydrine utilisables conformément à la présente invention pour la coloration des fibres capillaires sont les suivants :

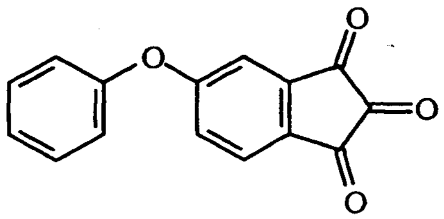

(a)

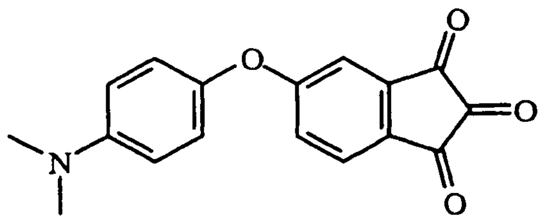

(b)

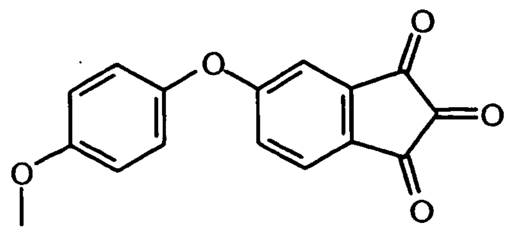

(c)

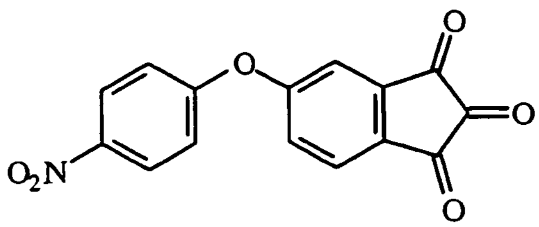

(d)

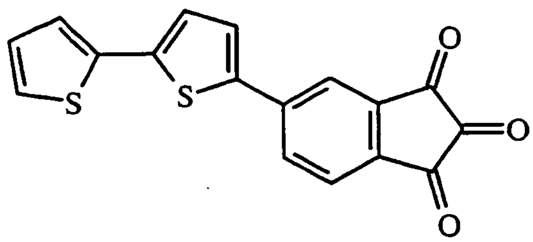

(e)

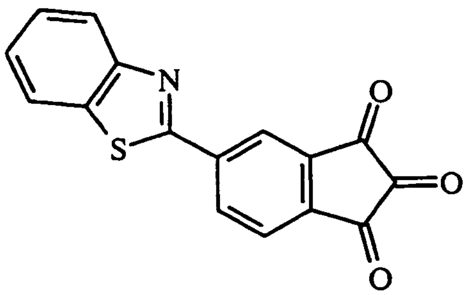

(f)

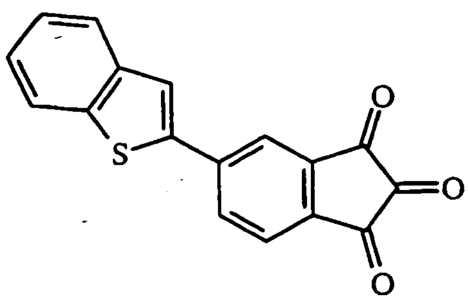

(g)

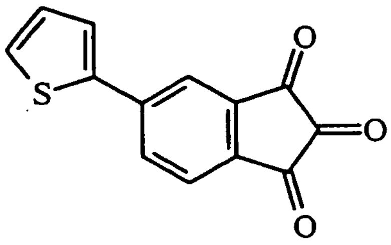

(h)

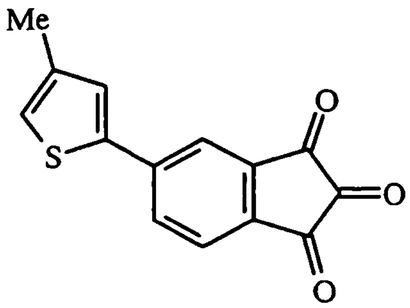

(i)

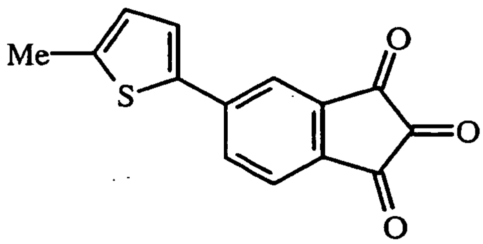

(j)

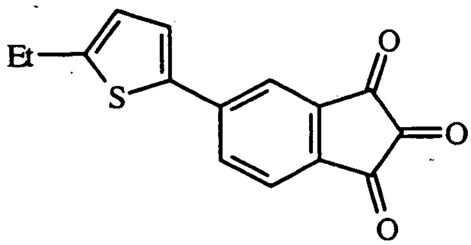

(k)

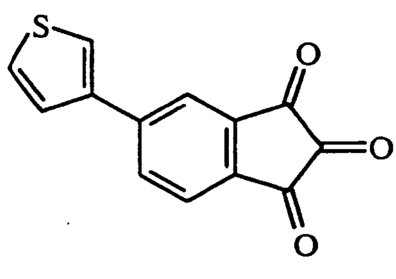

(l)

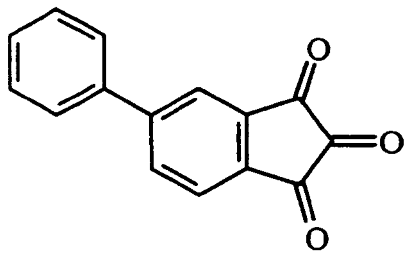
(m)

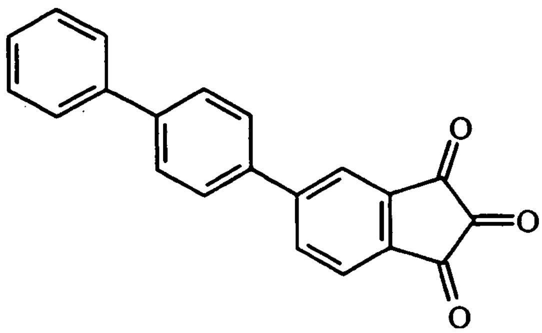
(n)

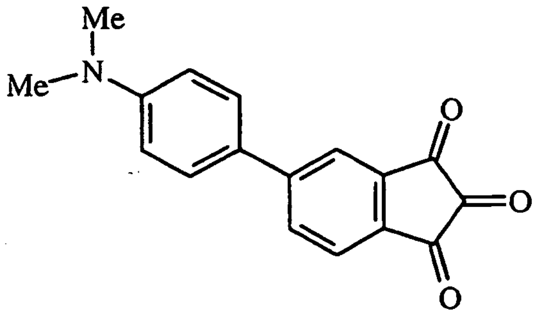
(o)

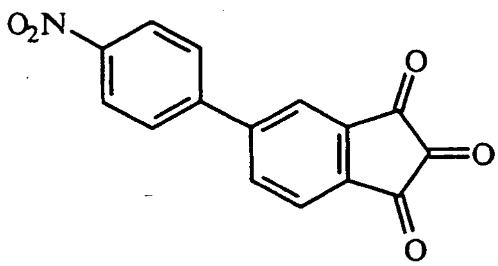
(p)

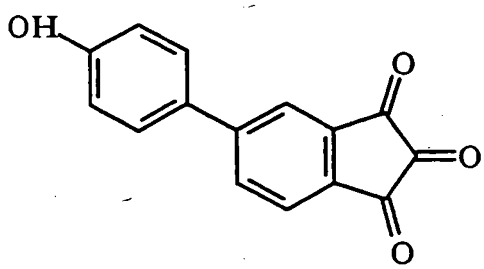
(q)

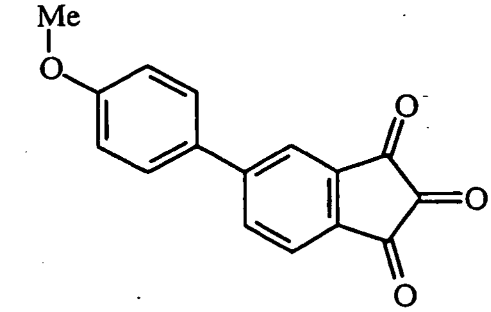

(r)

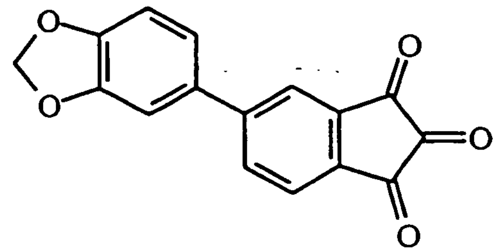

(s)

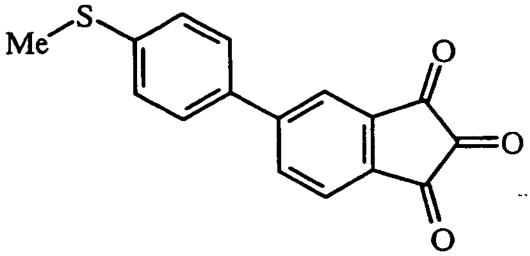

(t)

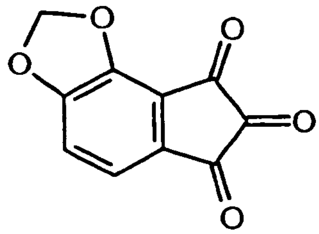

(u)

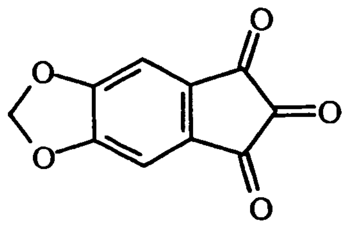

(v)

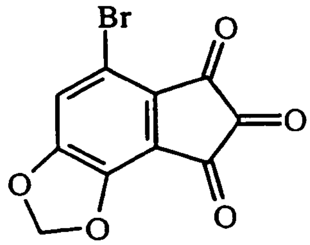

(w)

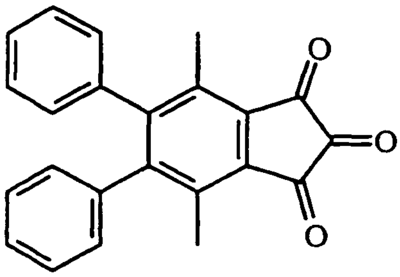

(x)

Les dérivés de ninhydrine utilisés dans la présente invention sont connus. La synthèse des dérives de ninhydrine (a) à (x) ci-dessus est décrite dans les publications suivantes :

(a) Kobus H. J., Pigou P. E., Della E. W., Taylor B., Davies P. J., Fingerprint Research in South Australia, in Almog J., Springer E., ed. Proceedings of the International Symposium on Fingerprint Detection and Identification, Ne'urim, Israel: Hemed Press, 1995, 227-230 ;
(b) Kobus H. J., Pigou P. E., Della E. W., Taylor B., Davies P. J., Fingerprint Research in South Australia, in Almog J., Springer E., ed. Proceedings of the International Symposium on Fingerprint Detection and Identification, Ne'urim, Israel: Hemed Press, 1995, 227-230 ;
(c) Della E. W., Janowski W. K., Pigou P. P., Taylor B. M., Synthesis of Fingerprint Reagents: Aromatic Nucleophilic Substitution as a Route to 5-substituted Ninhydrins, Synthesis, 1999, 12, 2119 - 2123 ;
(d) 2,2-dihydroxy-5-(4-nitro-phenoxy)-indane-1,3-dione : Kobus H. J., Pigou P. E., Della E. W., Taylor B., Davies P. J., Fingerprint Research in South Australia, in Almog J., Springer E., ed. Proceedings of the International Symposium on Fingerprint Detection and Identification, Ne'urim, Israel: Hemed Press, 1995, 227 -230 ;
(e) Hark R. R., Hauze D. B., Petrovskaïa O., Joullié M. M., Jahouari R., McComiskey P., Novel Approaches toward Ninhydrin Analogs, Tetrahedron Lett. 1994, 35, 7719 - 7722
(f) Hark R. R., Hauze D. B., Petrovskaïa O., Joullié M. M., Jahouari R., McComiskey P., Novel Approaches toward Ninhydrin Analogs, Tetrahedron Lett. 1994, 35, 7719 - 7722
(g) 5-benzo[b]thiophén-2-yl-2,2-dihydroxy-indane-1,3-dione : Hark R. R., Hauze D. B., Petrovskaïa O., Joullié M. M., Jahouari R., McComiskey P., Novel Approaches toward Ninhydrin Analogs, Tetrahedron Lett. 1994, 35, 7719 - 7722
(h) Hark R. R., Hauze D. B., Joullié M. M., Synthesis of Aryl-Substituted Ninhydrin Analogs, Abstracts of Papers, part 1, 204th National Meeting of the American Chemical Society, Washington, DC, Aug 23-28, 1992, American Society Washington DC, 1992, ANYL 055
(i) Hark R. R., Hauze D. B., Petrovskaïa O., Joullie M. M., Jahouari R., McComiskey P., Novel Approaches Toward Ninhydrin Analogs, Tetrahedron Lett. 1994, 35, 7719 - 7722
(j) Hark R. R., Hauze D. B., Petrovskaïa O., Joullié M. M., Jahouari R., McComiskey P., Novel Approaches toward Ninhydrin Analogs, Tetrahedron Lett. 1994, 35, 7719 - 7722
(k) Hark R. R., Hauze D. B., Petrovskaïa O., Joullié M. M., Jahouari R., McComiskey P., Novel Approaches toward Ninhydrin Analogs, Tetrahedron Lett. 1994, 35, 7719 - 7722
(l) Hark R. R., Hauze D. B., Petrovskaïa O., Joullié M. M., Jahouari R., McComiskey P., Novel Approaches toward Ninhydrin Analogs, Tetrahedron Lett. 1994, 35, 7719 - 7722
(m) Hark R. R., Hauze D. B., Joullié M. M., Synthesis of Aryl-Substituted Ninhydrin Analogs, Abstracts of Papers, part 1, 204th National Meeting of the American Chemical Society, Washington, DC, Aug 23-28, 1992, American Society Washington DC, 1992, ANYL 055

(n) Hark R. R., Hauze D. B., Joullié M. M., Synthesis of Aryl-Substituted Ninhydrin Analogs, Abstracts of Papers, part 1, 204th National Meeting of the American Chemical Society, Washington, DC, Aug 23-28, 1992, American Society Washington DC, 1992, ANYL 055

(o) Hark R. R., Hauze D. B., Petrovskaïa O., Joullié M. M., Jahouari R., McComiskey P., Novel Approaches toward Ninhydrin Analogs, Tetrahedron Lett. 1994, 35, 7719 - 7722

(p) Kobus H. J., Pigou P. E., Della E. W., Taylor B., Davies P. J., Fingerprint Research in South Australia, in Almog J., Springer E., ed. Proceedings of the International Symposium on Fingerprint Detection and Identification, Ne'urim, Israel: Hemed Press, 1995, 227-230

(q) Hauze D. B., Petrovskaïa O., Joullié M. M., Hark R. R., New Reagents for the Development of Fingerprint, in Almog J., Springer E., ed. Proceedings of the International Symposium on Fingerprint Detection and Identification, Ne'urim, Israel: Hemed Press, 1995, 119-123 :

(r) Della E. W., Janowski W. K., Pigou, P.P., Taylor B. M., Synthesis of Fingerprint Reagents, Aromatic Nucleophilic Substitution as a Route to 5-substituted Ninhydrins, Synthesis, 1999, 12, 2119-2123

(s) Hark R. R., Hauze D. B., Joullié M. M., Synthesis of Aryl-Substituted Ninhydrin Analogs, Abstracts of Papers, part 1, 204th National Meeting of the American Chemical Society, Washington, DC, Aug 23-28, 1992, American Society Washington DC, 1992, ANYL 055

(t) Della E. W., Janowski W. K., Pigou, P.P., Taylor B. M., Synthesis of Fingerprint Reagents, Aromatic Nucleophilic Substitution as a route to 5-substituted Ninhydrins, Synthesis, 1999, 12, 2119-2123

(u) Kametani T., Hibino S., Takano S., Studies on the Synthesis of Heterocyclic Compounds, part CDLIII Total Synthesis of (±)-ochrobirine, J. Chem. Soc., Perkin Trans. 1, 1972, 391-393

(v) 6,6-dihydroxy-indéno[5,6-d][1,3]dioxole-5,7-dione : Ruhemann S., Triketohydrindene Hydrate, Trans. Chem. Soc. 1910, 97, 2025-2031

(w) Nalliah B., Ahmed Q. A., Manske R. H. F., The Total Synthesis of (±)-ochrobirine, Can. J. Chem., 1972, 50, 1819 -1824

(x) Lennard C. J. Margot P. A. Stoilovic M., Warrener R. N. Synthesis of Ninhydrin Analogues and Their Application to Fingerprint Development : Preliminary results, J. Forens. Sci. Soc., 1986, 26, 323 - 328

Conformément à la présente invention, les dérivés de ninhydrine de formule (I) décrits ci-dessus peuvent être utilisés seuls pour la coloration des matières kératiniques. En effet, ces composés sont capables de générer des molécules colorées avec les fonctions amine de la kératine (réaction colorée).

On peut aussi utiliser conjointement des composés de formule (I) avec au moins un activateur qui permet de modifier la cinétique de réaction du composé ninhydrine avec la matière kératinique. Un tel activateur peut être un agent oxydant, un agent réducteur, des acides de brönsted, un catalyseur métallique tel que les catalyseurs à base de métal de transition tel que le fer, le platine, le palladium, des protéines notamment des enzymes, des composés modifiant la force ionique du milieu tels que des sels de NaCl, des composés à hydrogène labile choisi parmi ceux comportant une fonction amine primaire ou secondaire et ceux comportant une fonction méthylène activée. On peut, bien entendu, utiliser également un mélange de tels composés.

Les composés à fonction amine primaire ou amine secondaire sont de préférence des amines aromatiques.

On peut citer à titre d'exemples de telles amines aromatiques la N,N-diméthyl-p-phénylènediamine, la N,N-diéthyl-p-phénylènediamine, la N-(2-hydroxy-éthyl)-N-éthyl-p-phénylènediamine, la N,N-bis-(2-hydroxyéthyl)-p-phénylène-diamine, la N-(2-méthoxyéthyl)-p-phénylènediamine, la 2,3-, 2,4- ou 2,5-dichloro-p-phénylène-diamine, la 2-chloro-p-phénylènediamine, le dibromhydrate de 2,5-dihydroxy-4-morpholinoaniline, le 2-, 3- ou 4-aminophénol, le 2-aminométhyl-4-aminophénol, le 2-hydroxyméthyl-4-aminophénol, l'ortho-phénylènediamine, la p-phénylènediamine, l'ortho-toluènediamine, le 2,5-diaminotoluène, le 2,5-diaminophénol, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, la 4-méthylaminoaniline, la 3-amino-4-(2'-hydroxyéthyloxy)aniline, la 3,4-méthylènediaminoaniline, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 6-méthyl-3-amino-2-chlorophénol, le 2-méthyl-5-amino-4-chlorophénol, le 3,4-méthylènedioxyphénol, le 5-(2-hydroxyéthylamino)-4-méthoxy-2-méthylphénol, le 4-amino-2-hydroxyméthylphénol, le 1,3-diamino-2,4-diméthoxybenzène, l'acide 2-, 3-, 4-aminobenzoïque, l'acide 2-amino-, 3-amino ou 4-aminophénylacétique, l'acide 2,3-, 2,4-, 2,5-, 3,4- ou 3,5-diaminobenzoïque, l'acide 4-amino- ou 5-amino-salicylique, l'acide 3-amino-4-hydroxybenzoïque, l'acide 4-amino-3-hydroxybenzoïque, l'acide 2-amino-, 3-amino ou 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diaminopyrochatécol, le 4,6-diaminopyrogallol, le 3,5-diamino-4-hydroxypyrochatécol, et les anilines aromatiques et phénols aromatiques comportant un autre résidu aromatique, correspondant à la formule (II)

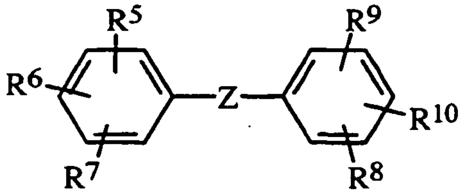

(II)

dans laquelle

$R^5$ représente un groupe hydroxy ou amino éventuellement substitués par un groupe alkyle en $C_{1-4}$, hydroxyalkyle en $C_{1-4}$ ou (alcoxy en $C_{1-4}$)-(alkyle en $C_{1-4}$),
$R^6$, $R^7$, $R^8$, $R^9$ et $R^{10}$ représentent chacun indépendamment un atome d'hydrogène, un groupe hydroxy ou un groupe amino, éventuellement substitués par un groupe alkyle en $C_{1-4}$, hydroxyalkyle en $C_{1-4}$ ou (alcoxy en $C_{1-4}$)-(alkyle en $C_{1-4}$), ou un groupe acide carboxylique ou sulfonique,
Z représente une liaison directe, une chaîne hydrocarbonée en $C_{1-4}$, saturée ou insaturée, éventuellement hydroxylée, un groupe carbonyle, sulfonyle ou imino, un atome d'oxygène ou de soufre, ou un groupe de formule $Q-(CH_2-P-CH_2-Q')_o$ où P représente une liaison directe ou un groupe $-CH_2-$ ou -CHOH-, Q et Q' représentent, indépendamment l'un de l'autre, un atome d'oxygène, un groupe $NR^{11}$ où $R^{11}$ représente un atome d'hydrogène, un groupe alkyle en $C_{1-4}$ ou hydroxyalkyle en $C_{1-4}$, ou un groupe $O-(CH_2)_pNH$ ou $NH-(CH_2)_{p'}-O$ où p et p' valent 2 ou 3 et o est un nombre entre 1 et 4.

Les amines primaires ou secondaires non-aromatiques sont par exemple le 2-aminoéthanol, la 2-méthoxyéthylamine, la 2-éthoxyéthylamine, le 2-(2-aminoéthoxy)-éthanol, le 2- ou 3-aminopropanol, la 2,3-dihydroxypropylamine, la 4-hydroxypropylamine, le 2-aminopropane-1,3-diol, le 2-amino-2-méthylpropanol, le 2-amino-2-méthylpropane-1,3-diol, le 2-amino-2-hydroxyméthylpropane-1,3-diol, la tétrahydropentylamine, les pentahydroxyhexylamines telles que la glucamine, la D-glucosamine, la D-galactosamine, le 1,2-diaminoéthane, le 1,2- ou 1,3-diaminopropane, le 1,3-diamino-2-propanol, la 2-(2-amnoéthylamino)-éthylamine, le 2-(2-aminoéthylamino)éthanol, la 3-(2-aminoéthylamino)-propylamine et le 3-(2-aminoéthylamino)propanol.

Les composés à fonction méthylène activée sont choisis par exemple parmi les suivants : l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline, l'iodure de 2,3-diméthylbenzothiazolium, le p-toluènesulfonate de 2,3-diméthyl-benzothiazolium, la rhodanine, l'acide rhodanine-3-acétique, l'iodure de 1-éthyl-2-quinaldinium, l'iodure de 1-méthyl-2-quinaldinium, l'acide barbiturique, l'acide thiobarbiturique, l'acide 1,3-diméthylthiobarbiturique, l'acide diéthylthiobarbiturique, l'oxindole, l'acétate de 3-indoxyle, la coumarone et la 1-méthyl-3-phényl-2-pyrazolinone.

Ces amines primaires et secondaires et ces composés à fonctions méthylène activée ainsi que d'autres composés à hydrogène labile sont décrits également dans les demandes de brevet DE 43 17 855, DE 197 17 222, DE 198 45 481 et DE 197 45 355 où ils sont utilisés pour la coloration des fibres kératiniques en combinaison avec des composés différents des dérivés de ninhydrine de formule (I).

Lorsque les dérivés de ninhydrine de formule (I) sont utilisés en combinaison avec une amine primaire ou secondaire ou avec un composé à fonction méthylène activée, il est nécessaire de conserver ces différents réactifs séparément pour éviter une réaction colorée prématurée. La mise en contact des réactifs ne se fait alors qu'immédiatement avant application sur les cheveux, par mélange extemporané de deux compositions contenant respectivement les dérivés de ninhydrine et les composés à hydrogène labile.

L'invention a par conséquent également pour objet un agent de coloration multi-composants comportant

- en tant que premier composant, une composition (a) contenant au moins un dérivé de ninhydrine de formule (I), et
- en tant que deuxième composant, une composition (b) contenant au moins un composé à fonction amine primaire ou secondaire ou au moins un composé à fonction méthylène activée, tels que décrits ci-dessus.

Cet agent de coloration multi-composants se présente de préférence sous forme d'un kit multi-compartiments, avec au moins un premier compartiment contenant le premier composant (composition (a)) et au moins un deuxième compartiment contenant le deuxième composant (composition (b)).

L'invention a également pour objet une composition cosmétique tinctoriale contenant au moins un dérivé de

ninhydrine de formule (I) et au moins un principe actif cosmétique.

Les principes actifs cosmétiques présents dans les compositions cosmétiques de la présente invention sont choisis par exemple parmi les vitamines, les saccharides, les oligosaccharides, les polysaccharides hydrolysés ou non, modifiés ou non, les acides aminés, les oligopeptides, les peptides, les protéines hydrolysées ou non, modifiées ou non, les polyacides aminés, les enzymes, les acides et alcools gras ramifiés ou non, les cires animales, végétales ou minérales, les céramides et les pseudo-céramides, les acides organiques hydroxylés, les filtres UV, les agents antioxydants et les agents anti-radicaux libres, les agents chélatants, les agents antipelliculaires, les agents régulateurs de séborrhée, les agents apaisants, les agents tensioactifs cationiques, anioniques, non-ioniques ou amphotères, les polymères cationiques, anioniques, neutres ou amphotères, les silicones organomodifiées ou non, les huiles minérales, végétales ou animales, les polyisobutènes et poly($\alpha$-oléfines), les esters gras, les polymères anioniques sous forme dissoute ou dispersée, les polymères non-ioniques sous forme dissoute ou dispersés, les agents réducteurs, les colorants capillaires tels que les colorants directs ou les précurseurs de coloration d'oxydation (bases et/ou coupleurs) différents des composés à fonction amine primaire ou secondaire revendiqués, les agents oxydants tels que le peroxyde d'hydrogène éventuellement associé à des persels, les pigments et leurs mélanges.

Le principe actif cosmétique est présent de préférence à raison de 0,001 à 50 % en poids, en particulier de 0,01 à 20 % en poids, et idéalement à raison de 0,1 à 10 % en poids, rapporté au poids total de la composition cosmétique.

Dans un mode de réalisation préféré de la composition cosmétique tinctoriale selon l'invention, le principe actif cosmétique est un agent tensioactif et/ou un agent polymérique (polymère), ces agents pouvant être de nature non-ionique, cationique, anionique ou amphotère.

Il ressort de ce qui précède que les compositions de coloration capillaire utilisées dans la présente invention sont stables au stockage lorsqu'elles contiennent, comme seuls réactifs, des dérivés de ninhydrine de formule (I) mais qu'elles doivent être préparées immédiatement avant emploi, lorsqu'elles contiennent à la fois des dérivés de ninhydrine de formule (I) et des composés à hydrogène labile tels que des amines primaires et secondaires ou des composés à fonction méthylène activée.

Ces compositions de coloration prêtes à l'emploi, qu'elles soient stables au stockage ou préparées immédiatement avant emploi, ont de préférence un pH compris entre 2 et 12, et en particulier entre 3 et 11.

Leur teneur en dérivés de ninhydrine de formule (I) est de préférence comprise entre 0,0001 % et 30 % en poids.

Les composés à hydrogène labile utilisés en combinaison avec les dérivés de ninhydrine de formule (I) sont de préférence présents à raison de 0,0001 à 30 %, rapporté au poids total de la composition.

La présente invention a en outre pour objet un procédé de coloration capillaire comprenant l'application, sur les cheveux, d'une composition de coloration capillaire prête à l'emploi telle que décrite ci-dessus. Cette composition est laissée en contact avec les fibres capillaires pendant un temps suffisant pour l'obtention de la coloration désirée. Ce temps de pose est généralement compris entre 5 minutes et 1 heure, et en particulier entre 15 et 30 minutes. La réaction colorée entre les dérivés de ninhydrine et les fonctions amine de la kératine ou les composés à hydrogène labile éventuellements présents, peut être accélérée par chauffage des cheveux imprégnées avec la composition de coloration. La température de chauffage ne dépasse de préférence pas 80 °C, et est en particulier inférieure ou égale à 60 °C.

Après obtention de la coloration souhaitée, les cheveux sont rincés et lavés.

Lorsqu'on utilise des composés à hydrogène labile tels que des amines primaires ou secondaires ou des composés à fonction méthylène activée, l'application des réactifs participant à la réaction colorée peut également se faire en deux temps, autrement dit on peut appliquer successivement deux compositions différentes contenant respectivement au moins un dérivé de ninhydrine de formule (I) et au moins un composé comportant une fonction amine primaire ou secondaire ou une fonction méthylène activée.

La présente invention a par conséquent également pour objet un procédé de coloration en deux temps comprenant le fait d'appliquer sur les cheveux l'une après l'autre, dans n'importe quel ordre, une composition (a) et une composition (b) telles que définies ci-dessus pour l'agent de coloration multi-composants.

Cette application séparée des deux compositions réactives présente l'avantage d'éviter la manipulation de compositions colorées et diminue ainsi les risques de souillure de matériaux tels que les vêtements.

La demanderesse a constaté que l'on obtenait également des colorations capillaires satisfaisantes lorsqu'une étape de rinçage intermédiaire était insérée entre l'application de la première composition et l'application de la deuxième composition.

De manière analogue à celle décrite ci-dessus, les cheveux imprégnés de composition (a) et/ou (b) peuvent être chauffés, de préférence jusqu'à une température de 80 °C, et en particulier jusqu'à une température ne dépassant pas 60 °C, un tel chauffage permettant d'accélérer la réaction colorée et de raccourcir le temps de pose.

## Exemple

On prépare la composition suivante :

| | |
|---|---|
| 5-benzo[b]thiophène-2-yl-2,2-dihydroxy-indane-1,3-dione | |
| (= hydrate du composé de formule (g)) | $10^{-2}$ moles |
| Ethanol | 50 g |
| NaOH | q.s.p pH 7 |
| Eau distillée | q.s.p. 100 g |

On applique cette composition sur deux mèches de cheveux naturels et permanentés à 90 % de cheveux blancs, de 1 g chacune. Le rapport de bain est de 5, le temps de pose de 30 minutes et la température de 60 °C. A la fin du temps de pose, les mèches sont rincées, puis lavées avec un shampooing standard.

L'intensité de coloration est évaluée par colorimétrie selon le système CIELAB à l'aide d'un colorimètre CM3600d de Minolta (illuminant D65, angle d'observation : 10 °, composante spéculaire incluse).

Le système de notation CIELAB définit un espace colorimétrique dans lequel chaque couleur est définie par 3 paramètres (L*, a* et b*) :

- le paramètre L* reflète *la clarté* de la couleur, la valeur de L* étant égale à 0 pour le noir et égale à 1 pour le blanc absolu. Plus la valeur de L* est élevée, moins la coloration est intense.
- le paramètre a* correspond à l'axe du couple antagoniste vert-rouge et le paramètre b* à l'axe du couple antagoniste bleu-jaune.

Le tableau ci-dessous montre les paramètres L*, a* et b* des mèches de cheveux naturels et de cheveux permanentés avant et après la montée de la couleur, ainsi que ΔE défini par l'équation ci-après :

$$\Delta E = \sqrt{(L^*_{final} - L^*_{initial})^2 + (a^*_{final} - a^*_{initial})^2 + (b^*_{final} - b^*_{initial})^2}.$$

ΔE reflète la variation globale de la couleur. Sa valeur est d'autant plus grande que la variation de couleur est importante.

| **Cheveux** | | **L*** | **a*** | **b*** | **ΔE** | **Couleur** |
|---|---|---|---|---|---|---|
| naturels | Avant coloration | 60,84 | 0,03 | 11,63 | - | - |
| naturels | Après coloration | 52,87 | -2,73 | 15,29 | 10,07 | verte |
| permanentés | Avant coloration | 61,78 | 0,33 | 12,74 | - | - |
| permanentés | Après coloration | 50,90 | -6,58 | 17,31 | 13,77 | verte |

## Revendications

1. Utilisation, pour la coloration de matières kératiniques, d'une composition contenant, dans un milieu approprié pour la teinture, au moins un composé de formule (I)

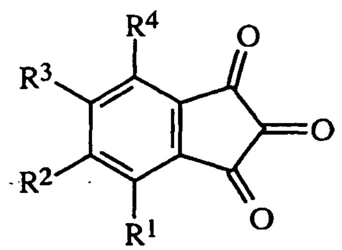

(I)

dans laquelle

$R^1$, $R^2$, $R^3$ et $R^4$ représentent, indépendamment les uns des autres, un atome d'hydrogène, un groupe carboxy (alkyle en $C_{1-6}$), (alkyle en $C_{1-6}$)-carboxy-(alkyle en $C_{1-6}$), aryloxy à 6 chaînons, hétéroaryloxy à 5 chaînons contenant un ou plusieurs hétéroatomes choisis parmi N, O, S et P, aryle comportant au moins 5 chaînons, monocyclique ou polycyclique, condensé ou non-condensé, contenant éventuellement un ou plusieurs hétéroatomes choisis parmi N, O, S et P, les groupes aryloxy, hétéroaryloxy, aryle ou hétéroaryle ci-dessus portant éventuellement un ou plusieurs substituants choisis parmi les atomes d'halogène, les groupes alkyle en $C_{1-9}$, hydroxy, alcoxy en C1-6, amino, mono- ou di-(alkyle en $C_{1-6}$)-amino, mono- ou di-(hydroxyalkyle en $C_{1-6}$)-amino, thio, alkylthio en $C_{1-6}$, thioalkyle en $C_{1-6,}$ (alkyle en C $_{1-6}$)-carbonyle, hydrogénocarbonyle, hydroxycarbonyle, (alcoxy en $C_{1-6}$)-carbonyle, nitro, sulfonato, tri(alkyle en $C_{1-6}$)-ammonio, imidazolyle et pyridinyle, ou les groupes protonés correspondants tels que ammonio, imidazolio ou pyridinio,
ou deux substituants voisins représentent ensemble un groupe $-O-CH_2-O-$,
avec la réserve qu'au moins un des symboles $R^1$ à $R^4$ représente un groupe différent d'un atome d'hydrogène, ou $R^1$ et $R^2$, $R^2$ et $R^3$ ou $R^3$ et $R^4$ représentent, ensemble, un groupe $-O-CH_2-O-$.

2. Utilisation selon la revendication 1, **caractérisée par le fait que** le noyau aryle, hétéroaryle, aryloxy ou hétéroaryloxy représenté par $R^1$, $R^2$, $R^3$ ou $R^4$ forme, avec le noyau indane, un système à électrons $\pi$ délocalisés.

3. Utilisation selon la revendication 1 ou 2, **caractérisée par le fait qu'**elle comprend en outre au moins un activateur qui permet de modifier la cinétique de réaction du composé ninhydrine avec la matière kératinique.

4. Utilisation selon la revendication 3, **caractérisée par le fait que** l'activateur est choisi parmi un agent oxydant, un agent réducteur, des acides de brönsted, un catalyseur métallique, des protéines, des composés modifiant la force ionique du milieu, des composés à hydrogène labile choisi parmi ceux comportant une fonction amine primaire ou secondaire et ceux comportant une fonction méthylène activée et un mélange de ceux-ci.

5. Utilisation selon la revendication 4, **caractérisée par le fait que** le composé comportant une fonction amine primaire ou secondaire est une amine aromatique choisie parmi la N,N-diméthyl-p-phénylènediamine, la N,N-diéthyl-p-phénylènediamine, la N-(2-hydroxy-éthyl)-N-éthyl-p-phénylènediamine, la N,N-bis-(2-hydroxyéthyl)-p-phénylène-diamine, la N-(2-méthoxyéthyl)-p-phénylènediamine, la 2,3-, 2,4- ou 2,5-dichloro-p-phénylène-diamine, la 2-chloro-p-phénylènediamine, le dibromhydrate de 2,5-dihydroxy-4-morpholinoaniline, le 2-, 3- ou 4-aminophénol, le 2-aminométhyl-4-aminophénol, le 2-hydroxyméthyl-4-aminophénol, l'ortho-phénylènediamine, la p-phénylènediamine, l'ortho-toluènediamine, le 2,5-diaminotoluène, le 2,5-diaminophénol, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, la 4-méthylaminoaniline, la 3-amino-4-(2'-hydroxyéthyloxy)aniline, la 3,4-méthylènediaminoaniline, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 6-méthyl-3-amino-2-chlorophénol, le 2-méthyl-5-amino-4-chlorophénol, le 3,4-méthylènedioxyphénol, le 5-(2-hydroxyéthylamino)-4-méthoxy-2-méthylphénol, le 4-amino-2-hydroxyméthylphénol, le 1,3-diamino-2,4-diméthoxybenzène, l'acide 2-, 3-, 4-aminobenzoïque, l'acide 2-amino-, 3-amino ou 4-aminophénylacétique, l'acide 2,3-, 2,4-, 2,5-, 3,4- ou 3,5-diaminobenzoïque, l'acide 4-amino- ou 5-amino-salicylique, l'acide 3-amino-4-hydroxybenzoïque, l'acide 4-amino-3-hydroxybenzoïque, l'acide 2-amino-, 3-amino ou 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diaminopyrochatécol, le 4,6-diaminopyrogallol, le 3,5-diamino-4-hydroxypyrochatécol, et les anilines aromatiques et phénols aromatiques comportant un autre résidu aromatique, correspondant à la formule (II)

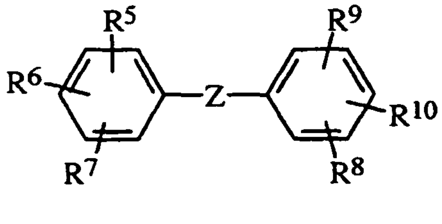

(II)

dans laquelle

$R^5$ représente un groupe hydroxy ou amino éventuellement substitués par un groupe alkyle en $C_{1-4}$, hydroxyalkyle en $C_{1-4}$, ou (alcoxy en $C_{1-4}$)-(alkyle en $C_{1-4}$),
$R^6$, $R^7$, $R^8$, $R^9$ et $R^{10}$ représentent chacun indépendamment un atome d'hydrogène, un groupe hydroxy ou un groupe amino, éventuellement substitués par un groupe alkyle en $C_{1-4}$, hydroxyalkyle en $C_{1-4}$ ou (alcoxy en $C_{1-4}$)-(alkyle en $C_{1-4}$), ou un groupe acide carboxylique ou sulfonique,
Z représente une liaison directe, une chaîne hydrocarbonée en $C_{1-4}$, saturée ou insaturée, éventuellement hydroxylée, un groupe carbonyle, sulfonyle ou imino, un atome d'oxygène ou de soufre, ou un groupe de formule $Q-(CH_2-P-CH_2-Q')_o$ où P représente une liaison directe ou un groupe $-CH_2-$ ou -CHOH-, Q et Q' représentent, indépendamment l'un de l'autre, un atome d'oxygène, un groupe $NR^{11}$ où $R^{11}$ représente un atome d'hydrogène, un groupe alkyle en $C_{1-4}$ ou hydroxyalkyle en $C_{1-4}$. ou un groupe $O-(CH_2)_pNH$ ou $NH-(CH_2)_{p'}-O$ où p et p' valent 2 ou 3 et o est un nombre entre 1 et 4

ou une amine aliphatique choisie parmi le 2-aminoéthanol, la 2-méthoxyéthylamine, la 2-éthoxyéthylamine, le 2-(2-aminoéthoxy)-éthanol, le 2- ou 3-aminopropanol, la 2,3-dihydroxypropylamine, la 4-hydroxypropylamine, le 2-aminopropane-1,3-diol, le 2-amino-2-méthylpropanol, le 2-amino-2-méthylpropane-1,3-diol, le 2-amino-2-hydroxyméthylpropane-1,3-diol, la tétrahydropentylamine, les pentahydroxyhexylamines telles que la glucamine, la D-glucosamine, la D-galactosamine, le 1,2-diaminoéthane, le 1,2- ou 1,3-diaminopropane, le 1,3-diamino-2-propanol, la 2-(2-amnoéthylamino)-éthylamine, le 2-(2-aminoéthylamino)éthanol, la 3-(2-aminoéthylamino)-propylamine et le 3-(2-aminoéthylamino)propanol.

6. Utilisation selon la revendication 4, **caractérisée par le fait que** le composé à fonction méthylène activée est choisi parmi l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline, l'iodure de 2,3-diméthylbenzothiazolium, le p-toluènesulfonate de 2,3-diméthyl-benzothiazolium, la rhodanine, l'acide rhodanine-acétique, l'iodure de 1-éthyl-2-quinaldinium, l'iodure de 1-méthyl-2-quinaldinium, l'acide barbiturique, l'acide thiobarbiturique, l'acide 1,3-diméthylthiobarbiturique, l'acide diéthylthiobarbitique, l'oxindole, l'acétate de 3-indoxyle, la coumarone et la 1-méthyl-3-phényl-2-pyrazinone.

7. Utilisation selon l'une quelconque des revendications précédentes, **caractérisée par le fait que** la composition a un pH compris entre 2 et 12, de préférence entre 3 et 11.

8. Utilisation selon l'une quelconque des revendications précédentes, **caractérisée par le fait que** la concentration du composé de formule (I) est comprise entre 0,0001 % et 30 %, rapporté au poids total de la composition.

9. Composition cosmétique tinctoriale contenant au moins un composé de formule (I) telle que définie dans la revendication 1 et un agent tensioactif et/ou un agent polymérique de nature non-ionique, cationique, anionique ou amphotère.

10. Composition cosmétique tinctoriale prête-à-l'emploi contenant au moins un composé de formule (I) telle que définie dans la revendication 1 et au moins un composé comportant une fonction amine primaire ou secondaire ou au moins un composé à fonction méthylène activée ou un mélange de ceux-ci. -

11. Agent de coloration multi-composants comportant

- en tant que premier composant, une composition (a) contenant au moins un composé de formule (I) tel que défini dans la revendication 1, et
- en tant que deuxième composant, une composition (b) contenant au moins un activateur tel que défini dans l'une quelconque des revendications 3 à 6.

12. Agent de coloration selon la revendication 11, **caractérisé par le fait qu'**il se présente sous forme d'un kit multi-compartiments, avec au moins un premier compartiment contenant la composition (a) et au moins un deuxième compartiment contenant la composition (b).

13. Procédé de coloration capillaire comprenant l'application, sur les cheveux, d'une composition de coloration capillaire selon l'une des revendications 9 ou 10, un temps de pose suffisant pour permettre l'obtention de la coloration souhaitée, puis le rinçage et le lavage des cheveux.

14. Procédé de coloration capillaire selon la revendication 13, **caractérisé par le fait qu'**il comprend le chauffage des

cheveux imprégnés de composition de coloration capillaire jusqu'à une température de 80 °C, de préférence de 60 °C.

**15.** Procédé de coloration capillaire comprenant le fait d'appliquer sur les cheveux l'une après l'autre, dans n'importe quel ordre, une composition (a) et une composition (b) telles que définies dans la revendication 11.

**16.** Procédé de coloration capillaire selon la revendication 15, **caractérisé par le fait qu'**une étape de rinçage intermédiaire est insérée entre l'application de la première composition et l'application de la deuxième composition.

**17.** Procédé de coloration capillaire selon l'une quelconque des revendications 15 ou 16, comprenant le chauffage des cheveux imprégnés avec la composition (a) et/ou (b) jusqu'à une température de 80 °C, de préférence de 60 °C.

## Claims

**1.** Use, for dyeing keratin materials, of a composition containing, in a medium appropriate for dyeing, at least one compound of formula (I)

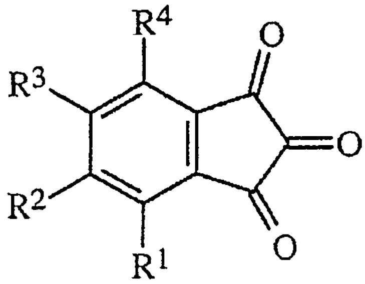

(I)

in which

$R^1$, $R^2$, $R^3$ and $R^4$ represent, independently of each other, a hydrogen atom, a carboxy($C_{1-6}$ alkyl) group, a ($C_{1-6}$ alkyl)carboxy($C_{1-6}$ alkyl) group, a 6-membered aryloxy group, a 5-membered heteroaryloxy group containing one or more heteroatoms chosen from N, O, S and P, an aryl group comprising at least 5 members, which is monocyclic or polycyclic, fused or non-fused, optionally containing one or more heteroatoms chosen from N, O, S and P, the above aryloxy, heteroaryloxy, aryl or heteroaryl groups optionally bearing one or more substituents chosen from halogen atoms, $C_{1-9}$ alkyl groups, hydroxyl groups, $C_{1-6}$ alkoxy groups, amino groups, mono- or di($C_{1-6}$ alkyl)amino groups, mono- or di($C_{1-6}$ hydroxyalkyl)amino groups, thio groups, $C_{1-6}$ alkylthio groups, $C_{1-6}$ thioalkyl groups, ($C_{1-6}$ alkyl)carbonyl groups, hydrogenocarbonyl groups, hydroxycarbonyl groups, ($C_{1-6}$ alkoxy)carbonyl groups, nitro groups, sulphonato groups, tri($C_{1-6}$ alkyl)ammonio groups, imidazolyl groups and pyridinyl groups, or the corresponding protonated groups such as ammonio, imidazolio or pyridinio, or two adjacent substituents represent together a group $-O-CH_2-O-$, with the proviso that at least one of the symbols $R^1$ to $R^4$ represents a group different from a hydrogen atom,
or $R^1$ and $R^2$, $R^2$ and $R^3$ or $R^3$ and $R^4$ represent together a group $-O-CH_2-O-$.

**2.** Use according to Claim 1, **characterized in that** the aryl, heteroaryl, aryloxy or heteroaryloxy ring represented by $R^1$, $R^2$, $R^3$ or $R^4$ forms, with the indan ring, a system of delocalized n electrons.

**3.** Use according to Claim 1 or 2, **characterized in that** it additionally comprises at least one activator which makes it possible to modify the kinetics of reaction of the ninhydrin compound with the keratinous material.

**4.** Use according to Claim 3, **characterized in that** the activator is chosen from an oxidizing agent, a reducing agent, Brönsted acids, a metal catalyst, proteins, compounds which modify the ionic strength of the medium, compounds containing a labile hydrogen chosen from those comprising a primary or secondary amine functional group and those comprising an activated methylene functional group and a mixture thereof.

**5.** Use according to Claim 4, **characterized in that** the compound comprising a primary or secondary amine functional group is an aromatic amine chosen from N,N-dimethyl-p-phenylenediamine, N,N-diethyl-p-phenylenediamine, N-(2-hydroxyethyl)-N-ethyl-p-phenylenediamine, N,N-bis-(2-hydroxyethyl)-p-phenylenediamine, N-(2-methoxyethyl)-p-phenylenediamine, 2,3-, 2,4- or 2,5-dichloro-p-phenylenediamine, 2-chloro-p-phenylenediamine, dibromohydrate of 2,5-dihydroxy-4-morpholinoaniline, 2-, 3- or 4-aminophenol, 2-aminomethyl-4-aminophenol, 2-hydroxymethyl-4-aminophenol, ortho-phenylenediamine, p-phenylenediamine, ortho-toluenediamine, 2,5-diaminotoluene, 2,5-diaminophenol, 2,5-diaminophenethol, 4-amino-3-methylphenol, 2-(2,5-diaminophenyl)ethanol, 2,4-diaminophenoxyethanol, 2-(2,5-diaminophenoxy)ethanol, 4-methylaminoaniline, 3-amino-4-(2'-hydroxyethyloxy)aniline, 3,4-methylenediaminoaniline, 3,4-methylenedioxyaniline, 3-amino-2,4-dichlorophenol, 4-methylaminophenol, 2-methyl-5-aminophenol, 3-methyl-4-aminophenol, 2-methyl-5-(2-hydroxyethylamino)phenol, 6-methyl-3-amino-2-chlorophenol, 2-methyl-5-amino-4-chlorophenol, 3,4-methylenedioxyphenol, 5-(2-hydroxyethylamino)-4-methoxy-2-methylphenol, 4-amino-2-hydroxymethylphenol, 1,3-diamino-2,4-dimethoxybenzene, 2-, 3-, 4-aminobenzoic acid, 2-amino-, 3-amino- or 4-aminophenylacetic acid, 2,3-, 2,4-, 2,5-, 3,4- or 3,5-diaminobenzoic acid, 4-amino- or 5-aminosalicylic acid, 3-amino-4-hydroxybenzoic acid, 4-amino-3-hydroxybenzoic acid, 2-amino-, 3-amino or 4-aminobenzenesulphonic acid, 3-amino-4-hydroxybenzenesulphonic acid, 4-amino-3-hydroxynaphthalene-1-sulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-triaminobenzene, 1,2,4-triaminobenzene, 1,2,4,5-tetraaminobenzene, 2,4,5-triaminophenol, pentaaminobenzene, hexaaminobenzene, 2,4,6-triaminoresorcinol, 4,5-diaminopyrocatechol, 4,6-diaminopyrogallol, 3,5-diamino-4-hydroxypyrocatechol, and aromatic anilines and aromatic phenols comprising another aromatic residue, corresponding to formula (II)

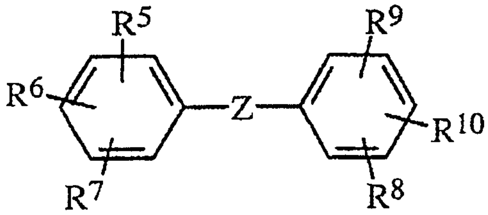 (II)

in which

$R^5$ represents a hydroxyl or amino group optionally substituted with a $C_{1-4}$ alkyl, $C_{1-4}$ hydroxyalkyl or ($C_{1-4}$ alkoxy) - ($C_{1-4}$ alkyl) group,
$R^6$, $R^7$, $R^8$, $R^9$ and $R^{10}$ each independently represent a hydrogen atom, a hydroxyl group or an amino group, optionally substituted with a $C_{1-4}$ alkyl, $C_{1-4}$ hydroxyalkyl or ($C_{1-4}$ alkoxy)-($C_{1-4}$ alkyl) group, or a carboxylic or sulphonic acid group,
Z represents a direct bond, a $C_{1-4}$ hydrocarbon chain which is saturated or unsaturated, optionally hydroxylated, a carbonyl, sulphonyl or imino group, an oxygen or sulphur atom, or a group of formula $Q-(CH_2P-CH_2-Q')_o$ where P represents a direct bond or a group $-CH_2-$ or -CHOH-, Q and Q' represent, independently of each other, an oxygen atom, a group $NR^{11}$ where $R^{11}$ represents a hydrogen atom, a $C_{1-4}$ alkyl or $C_{1-4}$ hydroxyalkyl group or a group $O-(CH_2)_pNH$ or $NH-(CH_2)_{p'}-O$ where p and p' are 2 or 3 and o is a number between 1 and 4 or an aliphatic amine chosen from 2-aminoethanol, 2-methoxyethylamine, 2-ethoxyethylamine, 2-(2-aminoethoxy) ethanol, 2- or 3-aminopropanol, 2,3-dihydroxypropylamine, 4-hydroxypropylamine, 2-aminopropane-1,3-diol, 2-amino-2-methylpropanol, 2-amino-2-methylpropane-1,3-diol, 2-amino-2-hydroxymethylpropane-1,3-diol, tetrahydropentylamine, pentahydroxyhexylamines such as glucamine, D-glucosamine, D-galactosamine, 1,2-diaminoethane, 1,2- or 1,3- diaminopropane, 1,3-diamino-2-propanol, 2-(2-aminoethylamino)ethylamine, 2-(2-aminoethylamino)ethanol, 3-(2-aminoethylamino)-propylamine and 3-(2-aminoethylamino)propanol.

6. Use according to Claim 4, **characterized in that** the compound containing an activated methylene functional group is chosen from 1,2,3,3-tetramethyl-3H-indolium iodide, 1,2,3,3-tetramethyl-3H-indolium p-toluenesulphonate, 1,2,3,3-tetramethyl-3H-indolium methanesulphonate, 1,3,3-trimethyl-2-methyleneindoline, 2,3-dimethylbenzothiazolium iodide, 2,3-dimethylbenzothiazolium p-toluenesulphonate, rhodanine, rhodanine acetic acid, 1-ethyl-2-quinaldinium iodide, 1-methyl-2-quinaldinium iodide, barbituric acid, thiobarbituric acid, 1,3-dimethylthiobarbituric acid, diethylthiobarbituric acid, oxindole, 3-indoxyl acetate, coumarone and 1-methyl-3-phenyl-2-pyrazinone.

7. Use according to any one of the preceding claims, **characterized in that** the composition has a pH of between 2 and 12, preferably between 3 and 11.

**8.** Use according to any one of the preceding claims, **characterized in that** the concentration of the compound of formula (I) is between 0.0001% and 30%, relative to the total weight of the composition.

**9.** Dyeing cosmetic composition containing at least one compound of formula (I) as defined in Claim 1 and a surfactant and/or a polymeric agent of nonionic, cationic, anionic or amphoteric nature.

**10.** Ready-to-use dyeing cosmetic composition containing at least one compound of formula (I) as defined in Claim 1 and at least one compound comprising a primary or secondary amine functional group or at least one compound comprising an activated methylene functional group or a mixture thereof.

**11.** Multicomponent dyeing agent containing

- as first component, a composition (a) containing at least one compound of formula (I) as defined in Claim 1, and
- as second component, a composition (b) containing at least one activator as defined in any one of Claims 3 to 6.

**12.** Dyeing agent according to Claim 11, **characterized in that** it is provided in the form of a multicompartment kit, with at least a first compartment containing the composition (a) and at least a second compartment containing the composition (b).

**13.** Hair dyeing method comprising the application, to the hair, of a hair dyeing composition according to either of Claims 9 and 10, a sufficient leave-in time to allow the desired colour to be obtained, and then rinsing and washing the hair.

**14.** Hair dyeing method according to Claim 13, **characterized in that** it comprises heating hair impregnated with hair dyeing composition to a temperature of 80°C, preferably of 60°C.

**15.** Hair dyeing method comprising the application, to the hair, one after the other, in any order, of a composition (a) and a composition (b) as defined in Claim 11.

**16.** Hair dyeing method according to Claim 15, **characterized in that** an intermediate rinsing step is inserted between the application of the first composition and the application of the second composition.

**17.** Hair dyeing method according to either of Claims 15 and 16, comprising heating hair impregnated with the composition (a) and/or (b) to a temperature of 80°C, preferably of 60°C.

**Patentansprüche**

**1.** Verwendung einer Zusammensetzung zum Färben von Keratinmaterialien, die in einem zum Färben geeigneten Medium mindestens eine Verbindung der Formel (I)

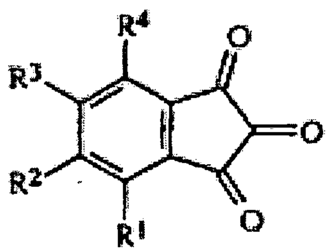

(I)

enthält,
in der bedeuten:

$R^1$, $R^2$, $R^3$ und $R^4$ unabhängig voneinander ein Wasserstoffatom, Carboxy-$C_{1-6}$-alkyl, $C_{1-6}$-Alkyl-carboxy-$C_{1-6}$-alkyl, Aryloxy mit 6 Ringgliedern, Heteroaryloxy mit 5 Ringgliedern, das ein oder mehrere Heteroatome enthält, die unter N, O, S und P ausgewählt sind, monocyclisches oder polycyclisches, kondensiertes oder nichtkondensiertes Aryl mit mindestens 5 Ringgliedern, das gegebenenfalls ein oder mehrere Heteroatome

enthält, die unter N, O, S und P ausgewählt sind, wobei die obigen Gruppen Aryloxy, Heteroaryloxy, Aryl oder Heteroaryl gegebenenfalls einen oder mehrere Substituenten aufweisen, die ausgewählt sind unter den Halogenatomen, $C_{1-9}$-Alkyl, Hydroxy, $C_{1-6}$-Alkoxy, Amino, Mono-$C_{1-6}$-alkylamino oder Di-($C_{1-6}$-alkyl)-amino, Monohydroxy-$C_{1-6}$-alkylamino oder Dihydroxy-$C_{1-6}$-alkylamino, Thio, $C_{1-6}$-Alkylthio, Thio-$C_{1-6}$-alkyl, $C_{1-6}$-Alkylcarbonyl, Hydrogencarbonyl, Hydroxycarbonyl, $C_{1-6}$-Alkoxycarbonyl, Nitro, Sulfonato, Tri-($C_{1-6}$-alkyl)-ammonio, Imidazolyl und Pyridinyl, oder den entsprechenden protonierten Gruppen, wie Ammonio, Imidazolio oder Pyridinio, oder zwei benachbarte Substituenten stellen zusammen eine Gruppe $_O_CH_2_O_$ dar,
mit der Maßgabe, dass mindestens eines der Symbole $R^1$ bis $R^4$ eine von einem Wasserstoffatom verschiedene Gruppe darstellt,
oder $R^1$ und $R^2$, $R^2$ und $R^3$ oder $R^3$ und $R^4$ bilden zusammen eine Gruppe $_O_CH_2_O_$.

2. Verwendung nach Anspruch 1, **dadurch gekennzeichnet, dass** der durch $R^1$, $R^2$, $R^3$ oder $R^4$ dargestellte Aryl-, Heteroaryl-, Aryloxy- oder Heteroaryloxy-Kern zusammen mit dem Indankern ein System mit delokalisierten $\pi$-Elektronen bildet.

3. Verwendung nach Anspruch 1 oder 2, **dadurch gekennzeichnet, dass** sie ferner mindestens einen Aktivator umfasst, der eine Modifizierung der Kinetik der Reaktion der Ninhydrinverbindung mit dem Keratinmaterial erlaubt.

4. Verwendung nach Anspruch 3, **dadurch gekennzeichnet, dass** der Aktivator ausgewählt ist unter einem Oxidationsmittel, einem Reduktionsmittel, Brönsted-Säuren, einem Metallkatalysator, Proteinen, Verbindungen, welche die Ionenstärke des Mediums modifizieren, Verbindungen mit labilem Wasserstoff, die ausgewählt sind unter Verbindungen mit einer primären oder sekundären Aminfunktion oder Verbindungen, die eine aktivierte Methylenfunktion aufweisen, sowie einem Gemisch dieser Verbindungen.

5. Verwendung nach Anspruch 4, **dadurch gekennzeichnet, dass** die Verbindung, die eine primäre oder sekundäre Aminfunktion aufweist, ein aromatisches Amin ist, das ausgewählt ist unter N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-, 2,4- oder 2,5-Dichlor-p-phenylendiamin, 2-Chlor-p-phenylendiamin, 2,5-Dihydroxy-4-morpholinoanilin-dihydrobromid, 2-, 3- oder 4-Aminophenol, 2-Aminomethyl-4-aminophenol, 2-Hydroxymethyl-4-aminophenol, o-Phenylendiamin, p-Phenylendiamin, o-Tolylendiamin, 2,5-Diaminotoluol, 2,5-Diaminophenol, 2,5-Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylaminoanilin, 3-Amino-4-(2'-hydroxyethyloxy)-anilin, 3,4-Methylendiaminoanilin, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)-phenol, 6-Methyl-3-amino-2-chlorphenol, 2-Methyl-5-amino-4-chlorphenol, 3,4-Methylendioxyphenol, 5-(2-Hydroxyethylamino)-4-methoxy-2-metylphenol, 4-Amino-2-hydroxymethylphenol, 1,3-Diamino-2,4-dimethoxybenzol, 2-, 3- oder 4-Aminobenzoesäure, 2-Amino-, 3-Amino- oder 4-Aminophenylessigsäure, 2,3-, 2,4-, 2,5-, 3,4- oder 3,5-Diaminobenzoesäure, 4-Amino- oder 5-Aminosalicylsäure, 3-Amino-4-hydroxybenzoesäure, 4-Amino-3-hydroxybenzoesäure, 2-Amino-, 3-Amino- oder 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin sowie den aromatischen Anilinen und aromatischen Phenolen, die einen weiteren aromatischen Rest aufweisen und der Formel (II) entsprechen,

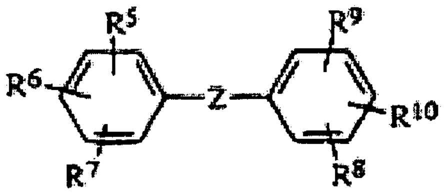 (II),

worin bedeuten:

$R^5$ eine Hydroxygruppe oder eine Aminogruppe, die gegebenenfalls mit einer $C_{1-4}$-Alkylgruppe, $C_{1-4}$-Hydro-

xyalkylgruppe oder einer $C_{1-4}$-Alkoxy-$C_{1-4}$-alkyl-Gruppe substituiert sind,
$R^6$, $R^7$, $R^8$, $R^9$ und $R^{10}$ jeweils unabhängig ein Wasserstoffatom, eine Hydroxygruppe oder eine Aminogruppe, die gegebenenfalls mit einer $C_{1-4}$-Alkylgruppe, $C_{1-4}$-Hydroxyalkylgruppe oder einer $C_{1-4}$-Alkoxy-$C_{1-4}$-alkyl-Gruppe substituiert sind, oder eine Carbonsäure- oder Sulfonsäuregruppe,
Z eine Einfachbindung, eine gesättigte oder ungesättigte und gegebenenfalls Hydroxylgruppen aufweisende $C_{1-4}$-Kohlenwasserstoffkette, Carbonyl, Sulfonyl oder Imino, ein Sauerstoffatom oder ein Schwefelatom oder eine Gruppe der Formel $Q_(CH_2_P_CH_2_Q')_o$, worin P eine Einfachbindung oder eine Gruppe $_CH_2_$ oder _CHOH_ bedeutet und Q und Q' unabhängig voneinander ein Sauerstoffatom, eine Gruppe $NR^{11}$, in der $R^{11}$ ein Wasserstoffatom, eine $C_{1-4}$-Alkylgruppe oder eine $C_{1-4}$-Hydroxyalkylgruppe bedeutet, oder eine Gruppe $O_(CH_2)_pNH$ oder $NH_(CH_2)_{p'}_O$ darstellen, worin p und p' 2 oder 3 bedeuten und o eine ganze Zahl von 1 bis 4 bedeutet,
oder ein aliphatisches Amin ist, das ausgewählt ist unter 2-Aminoethanol, 2-Methoxyethylamin, 2-Ethoxyethylamin, 2-(2-Aminoethoxy)-ethanol, 2- oder 3-Aminopropanol, 2,3-Dihydroxypropylamin, 4-Hydroxypropylamin, 2-Aminopropan-1,3-diol, 2-Amino-2-methylpropanol, 2-Amino-2-methylpropan-1,3-diol, 2-Amino-2-hydroxymethylpropan-1,3-diol, Tetrahydropentylamin, Pentahydroxyhexylaminen, wie Glucamin, D-Glucosamin, D-Galactosamin, 1,2-Diaminoethan, 1,2- oder 1,3-Diaminopropan, 1,3-Diamino-2-propanol, 2-(2-Aminoethylamino)-ethylamin, 2-(2-Aminoethylamino)-ethanol, 3-(2-Aminoethylamino)-propylamin und 3-(2-Aminoethylamino)-propanol.

6. Verwendung nach Anspruch 4, **dadurch gekennzeichnet, dass** die Verbindung mit einer aktivierten Methylenfunktion ausgewählt ist unter 1,2,3,3,-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin, 2,3-Dimethylbenzothiazoliumiodid, 2,3-Dimethylbenzothiazolium-p-toluolsulfonat, Rhodamin, Rhodaminessigsäure, 1-Ethyl-2-chinaldiniumiodid, 1-Methyl-2-chinaldiniumiodid, Barbitursäure, Thiobarbitursäure, 1,3-Dimethylthiobarbitursäure, Diethylthiobarbitursäure, Oxindol, 3-Indoxylacetat, Cumaron und 1-Methyl-3-phenyl-2-pyrazinon.

7. Verwendung nach einem der vorhergehenden Ansprüche, **dadurch gekennzeichnet, dass** die Zusammensetzung einen pH-Wert von 2 bis 12 und vorzugsweise von 3 bis 11 aufweist.

8. Verwendung nach einem der vorhergehenden Ansprüche, **dadurch gekennzeichnet, dass** die Konzentration der Verbindung der Formel (I) im Bereich von 0,0001 % bis 30 % liegt, bezogen auf das Gesamtgewicht der Zusammensetzung.

9. Kosmetische Zusammensetzung zum Färben, die mindestens eine Verbindung der Formel (I) wie in Anspruch 1 definiert sowie ein nichtionisches, kationisches, anionisches oder amphoteres Tensid und/oder ein nichtionisches, kationisches, anionisches oder amphoteres polymeres Mittel enthält.

10. Anwendungsfertige kosmetische Zusammensetzung zum Färben, die mindestens eine Verbindung der Formel (I) wie in Anspruch 1 definiert und mindestens eine Verbindung mit einer primären oder sekundären Aminfunktion oder mindestens eine Verbindung mit einer aktivierten Methylenfunktion oder ein Gemisch dieser beiden Verbindungen enthält.

11. Mehrkomponentiges Färbemittel, das enthält:

- als erste Komponente eine Zusammensetzung (a), die mindestens eine Verbindung der Formel (I) wie in Anspruch 1 definiert enthält, und
- als zweite Komponente eine Zusammensetzung (b), die mindestens einen Aktivator wie in einem der Ansprüche 3 bis 6 definiert enthält.

12. Färbemittel nach Anspruch 11, **dadurch gekennzeichnet, dass** es in Form eines Kits mit mehreren Compartments vorliegt, bei dem mindestens ein erstes Compartment die Zusammensetzung (a) und mindestens ein zweites Compartment die Zusammensetzung (b) enthält.

13. Verfahren zur Haarfärbung, das die Anwendung einer Haarfärbezusammensetzung nach Anspruch 9 oder 10 auf die Haare, eine Einwirkungsdauer, die zur Erzielung der gewünschten Färbung ausreicht, anschließendes Spülen und das Waschen der Haare umfasst.

14. Verfahren zur Haarfärbung nach Anspruch 13, **dadurch gekennzeichnet, dass** es das Erwärmen der mit der

Haarfärbezusammensetzung imprägnierten Haare bis auf eine Temperatur von 80° C und bevorzugt von 60° C umfasst.

**15.** Verfahren zur Haarfärbung, das die Anwendung einer Zusammensetzung (a) und einer Zusammensetzung (b), wie sie in Anspruch 11 definiert sind, nacheinander in einer beliebigen Reihenfolge auf die Haare umfasst.

**16.** Verfahren zur Haarfärbung nach Anspruch 15, **dadurch gekennzeichnet, dass** eine Stufe des Zwischenspülens zwischen die Anwendung der ersten Zusammensetzung und die Anwendung der zweiten Zusammensetzung eingeschoben wird.

**17.** Verfahren zur Haarfärbung nach Anspruch 15 oder 16, welches das Erwärmen der mit der Zusammensetzung (a) und/oder (b) imprägnierten Haare bis auf eine Temperatur von 80° C und bevorzugt von 60° C umfasst.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- DE 4317855
- DE 19717222
- DE 19845481
- DE 19745355


### Littérature non-brevet citée dans la description


- Fingerprint Research in South Australia. **KOBUS H. J ; PIGOU P. E ; DELLA E. W ; TAYLOR B ; DAVIES P. J.** Proceedings of the International Symposium on Fingerprint Detection and Identification. Hemed Press, 1995, 227-230
- **DELLA E. W ; JANOWSKI W. K ; PIGOU P. P ; TAYLOR B. M.** Synthesis of Fingerprint Reagents. *Aromatic Nucleophilic Substitution as a Route to 5-substituted Ninhydrins, Synthesis,* 1999, vol. 12, 2119-2123
- Fingerprint Research in South Australia. **KOBUS H. J ; PIGOU P. E ; DELLA E. W ; TAYLOR B ; DAVIES P. J.** Proceedings of the International Symposium on Fingerprint Detection and Identification. Hemed Press, 1995, 227-230
- **HARK R. R ; HAUZE D. B ; PETROVSKAÏA O ; JOULLIÉ M. M ; JAHOUARI R ; MCCOMISKEY P.** Novel Approaches toward Ninhydrin Analogs. *Tetrahedron Lett.,* 1994, vol. 35, 7719-7722
- **HARK R. R ; HAUZE D. B ; JOULLIÉ M. M.** Synthesis of Aryl-Substituted Ninhydrin Analogs. *204th National Meeting of the American Chemical Society,* 23 Août 1992
- **HARK R. R ; HAUZE D. B ; PETROVSKAÏA O ; JOULLIE M. M ; JAHOUARI R ; MCCOMISKEY P.** Novel Approaches Toward Ninhydrin Analogs. *Tetrahedron Lett.,* 1994, vol. 35, 7719-7722
- Fingerprint Research in South Australia. **KOBUS H. J ; PIGOU P. E. ; DELLA E. W ; TAYLOR B ; DAVIES P. J.** Proceedings of the International Symposium on Fingerprint Detection and Identification. Hemed Press, 1995, 227-230
- New Reagents for the Development of Fingerprint. **HAUZE D. B ; PETROVSKAÏA O ; JOULLIÉ M. M ; HARK R. R.** Proceedings of the International Symposium on Fingerprint Detection and Identification. Hemed Press, 1995, 119-123
- **DELLA E. W ; JANOWSKI W. K ; PIGOU, P.P ; TAYLOR B. M.** Synthesis of Fingerprint Reagents, Aromatic Nucleophilic Substitution as a Route to 5-substituted Ninhydrins. *Synthesis,* 1999, vol. 12, 2119-2123
- **DELLA E. W ; JANOWSKI W. K ; PIGOU, P.P ; TAYLOR B. M.** Synthesis of Fingerprint Reagents, Aromatic Nucleophilic Substitution as a route to 5-substituted Ninhydrins. *Synthesis,* 1999, vol. 12, 2119-2123
- **KAMETANI T ; HIBINO S ; TAKANO S.** Studies on the Synthesis of Heterocyclic Compounds, part CDLII Total Synthesis of (±)-ochrobirine. *J. Chem. Soc., Perkin Trans,* 1972, vol. 1, 391-393
- **RUHEMANN S.** Triketohydrindene Hydrate. *Trans. Chem. Soc,* 1910, vol. 97, 2025-2031
- **NALLIAH B ; AHMED Q. A ; MANSKE R. H. F.** The Total Synthesis of (±)-ochrobirine. *Can. J. Chem,* 1972, vol. 50, 1819-1824
- **LENNARD C. J ; MARGOT P. A ; STOILOVIC M ; WARRENER R. N.** Synthesis of Ninhydrin Analogues and Their Application to Fingerprint Development : Preliminary results. *J. Forens. Sci. Soc,* 1986, vol. 26, 323-328